# FASCICULE DE BREVET EUROPEEN

(11) **EP 0 332 523 B1**
(45) Date de publication et mention de la délivrance du brevet: **13.01.1993**
(21) Numéro de dépôt: 89400621.2
(22) Date de dépôt: 06.03.1989
(51) Int. Cl.: C12N 15/00, A61K 37/02, C12P 21/02

(54) **Variants de l'hirudine, leurs utilisations et les procédés pour les obtenir**
Hirudin-Varianten, deren Verwendung und Verfahren zu deren Herstellung
Hirudin variants, their use and process for their preparation

(30) Priorité: 08.03.1988 FR 8802925
(43) Date de publication de la demande: 13.09.1989
(73) Titulaire: TRANSGENE S.A., 67082 Strasbourg Cédex (FR)
(72) Inventeur: Courtney, Michael, F-67170 Geudertheim (FR); Degryse, Eric, F-67000 Strasbourg (FR); Loison, Gérard, F-31300 Toulouse (FR)
(74) Mandataire: Warcoin, Jacques

(56) Documents cités:
- EP-A- 0 158 564
- EP-A- 0 171 024
- EP-A- 0 273 800

## Description

La présente invention concerne des variants de l'hirudine, leurs utilisations et les procédés pour les obtenir.

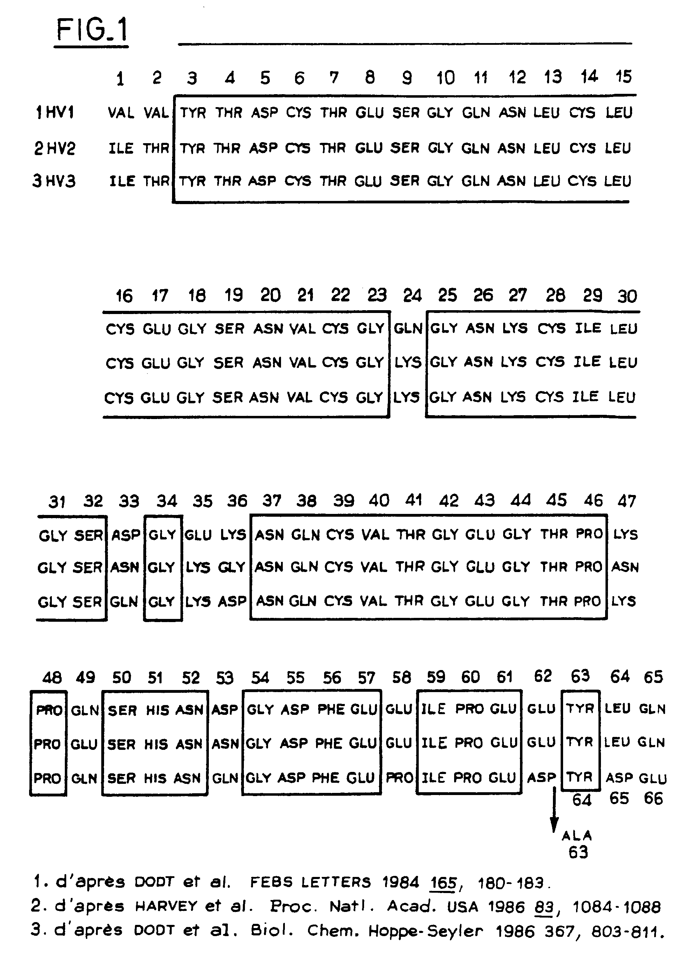

L'hirudine naturelle est un mélange de peptides de 65 et 66 acides aminés sécrétés en très petite quantité par les glandes salivaires de la sangsue médicinale (Markwardt 1970 ; Petersen et al. 1976 ; Chang 1983 ; Dodt et al. 1984, 1985, 1986 ; Harvey et al. 1986).
Le premier variant décrit, HV1, correspond à l'hirudine qu'on isole du corps de la sangsue ; le deuxième, HV2 (Harvey et al. 1986) diffère du premier par 9 acides aminés, le troisième (Dodt et al. 1986) est identique à HV2 jusqu'à la sérine 32 mais diffère par 10 acides aminés dans la partie C-terminale, qui comprend en outre un acide aminé supplémentaire (Ala⁶³). Ce troisième variant est désigné par HV3. Les structures de ces trois variants sont décrites sur la figure 1.

Les séquences de ces variants naturels comportent 65 ou 66 acides aminés et peuvent être considérées comme deux domaines : une partie N-terminale globulaire qui contient 3 ponts disulfures et une partie C-terminale acide qui présente une homologie avec le site de clivage par la thrombine dans la molécule de prothrombine. Cette homologie a permis de penser que la région qui entoure la position 47 pouvait être un site de fixation de l'hirudine sur la thrombine. C'est pourquoi la Demanderesse a déjà décrit, dans la demande de brevet européen n° 87402696.6 des variants de l'hirudine qui comportent un acide aminé différent de l'acide aminé existant dans la forme naturelle en position 47. En particulier, le variant HV2 (Lys^{4/}) a été décrit comme présentant des cinétiques d'inhibition de la thrombine améliorées et une activité antithrombotique plus grande que la molécule non-modifiée.

La présente invention a pour objet de nouveaux variants de l'hirudine présentant des propriétés biologiques améliorées.

Tout d'abord, afin d'améliorer le profil pharmacocinétique de la molécule d'hirudine pour prolonger son action in vivo et ainsi diminuer la dose thérapeutique nécessaire, on peut introduire des modifications post-traductionnelles covalentes, en particulier des chaînes latérales hydrocarbonées qui peuvent masquer les sites du polypeptide responsables de l'élimination de la molécule de la circulation.

C'est pourquoi on crée sur la molécule un ou plusieurs sites de glycosylation susceptibles de favoriser l'addition des chaînes hydrocarbonées. La glycosylation s'effectue souvent sur des résidus Asn en des sites spécifiques de reconnaissance Asn-X-Thr ou Asn-X-Ser et sur l'hirudine, une modification possible consiste à remplacer l'acide aminé Lys³⁵ par l'acide aminé Thr³⁵, ce qui conduit à la séquence Asn³³-Gly³⁴-Thr³⁵.

D'autre part, l'hirudine naturelle ne possède pas de sites spécifiques de reconnaissance par les récepteurs de surface des cellules sanguines. Pour modifier le mode d'action in vivo de l'hirudine, il a paru intéressant d'introduire dans la molécule d'hirudine une séquence Arg-Gly-Asp-Ser (RGDS) pour permettre une meilleure interaction avec les récepteurs de surface des cellules. Cette séquence RGDS est présente dans le fibrinogène sur un site impliqué dans la liaison avec le récepteur plaquettaire GpIIb/IIIa. Des travaux récents (Ruggeri et al., 1986) ont pu démontrer que l'interaction fibrinogène, GpIIb/IIIa était nécessaire pour l'agrégation plaquettaire et que les peptides comportant une séquence RGDS inhibaient l'agrégation plaquettaire.

Enfin, dans les cas où l'hirudine est préparée par les techniques du génie génétique, notamment par expression et sécrétion à partir de la levure Saccharomyces cerevisiae, on a constaté qu'au cours de sa production l'hirudine pouvait subir des dégradations indésirables à l'extrémité C-terminale sous l'effet de carboxypeptidases. Pour réduire ces dégradations l'invention propose de modifier l'extrémité C-terminale de la molécule en lui ajoutant un ou plusieurs acides aminés, par exemple la proline.

L'invention a donc pour objet des variants de l'hirudine, caractérisés en ce que la structure peptidique de ces variants comporte, par rapport aux variants de base HV, une des modifications suivantes :
- dans au moins un site Asn-X-Y de HV où X est un acide aminé quelconque, Y est choisi parmi Ser ou Thr ;
- une séquence Arg-Gly-Asp-Ser remplace une séquence d'acides aminés ;
- au moins un acide aminé supplémentaire est ajouté à l'extrémité C-terminale ;

les variants de base HV sont choisis parmi :
HV1, HV2, HV3, HV2 (Val¹, Val²), HV1 (AA⁶³), HV2 (AA⁶³), HV3 (AA⁶⁴), HV2 (Val¹ , Val², AA⁶³), HV2 (Lys⁴⁷), HV2 (Val¹, Val², Lys⁴⁷), HV2 (Lys⁴⁷, AA⁶³), HV2 (Val¹, Val², Lys⁴⁷, AA⁶³), et AA est choisi parmi Glu et Asp.

Les variants de base peuvent être obtenus selon des procédés décrits dans la demande de brevet européenne 87.402696.6 au nom de la Demanderesse.

L'invention concerne en particulier une famille de molécules hybrides dans lesquelles HV est HV2 (Lys⁴⁷) et qui, en plus d'au moins l'une des modifications précédentes, ou comme seule modification, comportent à la place des acides aminés Ile¹-Thr² les acides aminés Val¹-Val².

Il s'agit en particulier des variants de : HV2 (Lys⁴⁷), HV2 (Lys⁴⁷, AA⁶³), HV2 (Val¹, Val², Lys⁴⁷), HV2 (Val¹, Val², Lys⁴⁷, AA⁶³).

L'un des sites plus particulièrement utilisable pour effectuer la première modification se situe au niveau des acides aminés 33 à 35 de HV2, en remplaçant (Lys³⁵) par (Thr³⁵) ou (Ser³⁵).

L'un des sites plus particulièrement utilisable pour effectuer la seconde modification se situe sur les acides aminés 33 à 36 et les variants (Arg³³, Asp³⁵, Ser³⁶) sont les plus intéressants, en particulier le variant de HV2 (Lys⁴⁷).

Bien qu'il soit possible d'ajouter un acide aminé quelconque à l'extrémité C-terminale, on préfère utiliser la proline, par exemple le variant HV2 (Lys⁴⁷, Pro⁶⁶).

Enfin, pour obtenir une plus forte activité inhibitrice de la thrombine, des propriétés pharmacocinétiques améliorées, une plus grande stabilité et une action antithrombotique améliorée par rapport au variant HV2 (Lys⁴⁷) qui a été décrit par la Demanderesse dans la demande de brevet européen n° 87402696.6, les variants suivants présentent un grand intérêt : 
A B TYR THR ASP CYS THR GLU SER GLY GLN ASN LEU CYS LEU
CYS GLU GLY SER ASN VAL CYS GLY LYS GLY ASN LYS CYS ILE LEU
GLY SER ASN GLY LYS GLY ASN GLN CYS VAL THR GLY GLU GLY THR
PRO LYS PRO GLU SER HIS ASN ASN GLY ASP PHE GLU GLU ILE PRO
GLU GLU C LEU GLN
dans laquelle A-B représente Ile-Thr ou Val-Val et C représente Glu ou Asp.

En effet, la sulfatation de la tyrosine 63 constitue peut-être une différence importante entre l'hirudine naturelle et l'hirudine obtenue par recombinaison génétique, comme le suggéraient les études cinétiques de Stone et Hofsteenge (1986).

C'est pourquoi, dans les dérivés précédents, la (Tyr⁶³) a été remplacée par Glu ou Asp.

Les modifications précédentes peuvent se cumuler.
Parmi les variants en cause, il faut citer de préférence:
HV2 (Lys⁴⁷, Asp⁶³)
HV2 (Lys⁴⁷, Asn³³-Gly³⁴-Thr³⁵)
HV2 (Lys⁴⁷, Arg³³, Asp³⁵, Ser³⁶)
HV2 (Lys⁴⁷, Pro⁶⁶)
ainsi que les variants :
(Val¹-Val²) HV2 (Lys⁴⁷)
(Val¹-Val²) HV2 (Lys⁴⁷, Asp⁶³)
(Val¹-Val²) HV2 (Lys⁴⁷, Thr³⁵)
(Val¹-Val²) HV2 (Lys⁴⁷, Arg³³, Asp³⁵, Ser³⁶)
(Val¹-Val²) HV2 (Lys⁴⁷, Pro⁶⁶).
Les différents variants qui ont conservé la Tyr⁶³ peuvent être utilisés sous forme sulfatée ou non. Cette sulfatation peut être obtenue chimiquement ou par voie biologique.

La présente invention s'étend aux procédés biologiques et/ou chimiques permettant de préparer les variants mentionnés ci-dessus.

On peut en effet obtenir ces variants par synthèse, hémisynthèse et/ou par les techniques connues du génie génétique.

Ainsi par exemple le clonage et l'expression de la séquence codant pour HV2 et l'obtention de l'hirudine correspondante à partir de levures ont été décrits dans la publication européenne de brevet EP.A.200 655.

Les variants selon l'invention peuvent être obtenus par des techniques équivalentes après avoir modifié la séquence codant pour HV2 par exemple par mutagénèse dirigée.

En particulier par mutagénèse dirigée in vitro on a construit des variants HV2 où l'asparagine 47 est remplacée par une lysine et où soit la lysine 35 est remplacée par une thréonine, soit l'asparagine 33 est remplacée par une arginine, la lysine 35 par un aspartate et la glycine 36 par une sérine, soit la tyrosine 63 est remplacée par un aspartate soit encore la séquence Ile¹ Thr² est remplacée par Val¹-Val².

En particulier, il est possible d'utiliser des blocs d'expression fonctionnels dans la levure tels que décrits dans la demande de brevet EP-A-200 655 et dans lesquels la séquence de l'hirudine code pour les variants précédents ; ces blocs fonctionnels d'ADN peuvent être portés par un plasmide vecteur.

Pour diriger l'expression et la sécrétion par la levure des gènes correspondant aux différents variants, ceux-ci sont intégrés dans un vecteur de levure qui comprend, de préférence, les éléments suivants, qui ont été décrits dans la demande EP-A-200 655 :
- l'origine de réplication du plasmide de levure 2 µ,
- le gène ura3,
- une origine de réplication dans E. coli et un marqueur de résistance à un antibiotique,
- un promoteur de transcription, la séquence "leader" et la séquence pre-pro du précurseur du facteur alpha, fusionnée en phase, en amont de la séquence codante du variant de l'hirudine,
- le terminateur de transcription du gène PGK de la levure, qui sera placé en aval du gène dudit variant.

L'invention concerne également les levures transformées par ces vecteurs ou par ce bloc fonctionnel d'ADN et leur application à la préparation des variants de l'hirudine.

En particulier, l'invention concerne un procédé de préparation d'un variant de l'hirudine par fermentation d'une levure selon l'invention et récupération de l'hirudine produite dans le milieu de culture sous forme mature ou sous forme d'un précurseur maturable in vitro.

Les techniques mises en oeuvre ont déjà été décrites plus en détail dans les demandes de brevet EP-A-200 655 et EP-87 401649.6.

Les variants de l'hirudine ainsi obtenus peuvent être utilisés comme cela est décrit dans la demande de brevet EP-A-200 655 en tant qu'inhibiteur de la thrombine, tant in vivo que in vitro.

En particulier, ces variants peuvent être utilisés dans des compositions pharmaceutiques, seuls ou en combinaison avec d'autres principes actifs, ou bien dans le cadre de tests ou de diagnostic, in vitro ou in vivo. Dans ce dernier cas, il peut être intéressant de marquer les variants, par exemple par un marquage radioactif, fluorescent, enzymatique ou autre.

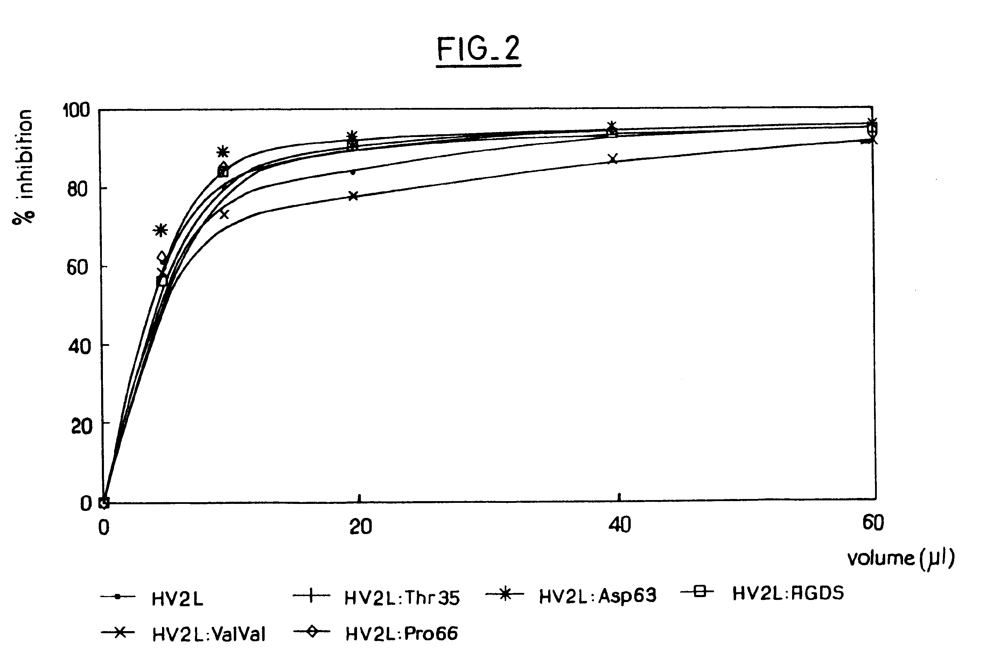

La présente invention est illustrée par les exemples suivants à l'aide de la figure 1 qui représente les séquences des 3 variants naturels de l'hirudine et de la figure 2 qui représente le pourcentage d'inhibition de l'activité protéolytique de la thrombine sur le chromozyme en fonction des volumes de surnageants des cultures de levure produisant les variants de l'hirudine HV2 (Lys⁴⁷), notés HV2L.

### Exemple 1 Obtention de gènes mutés codant pour des variants de l'hirudine HV2 (Lys⁴⁷), par mutagénèse dirigée in vitro.

Pour effectuer une mutagénèse dirigée in vitro, le fragment d'ADN que l'on veut modifier est cloné dans la forme réplicative d'un phage M13 simple brin ; le génome du phage recombinant est isolé et mis à hybrider avec un oligonucléotide synthétique qui porte la séquence mutée. Cet oligonucleotide sert d'amorce à la synthèse du brin complémentaire et l'ADN rendu ainsi double brin est utilisé pour transformer une bactérie réceptrice qui va produire du phage portant la mutation recherchée (Zoller and Smith, 1983).

La séquence codante de l'hirudine HV2 a été manipulée pour en faire une "cassette d'expression" fonctionnelle dans la levure et pour assurer la sécrétion de la protéine synthétisée. Pour cela, un segment d'ADN codant pour la séquence pre-pro de l'α-phéromone de levure a été placé en amont du gène. Cette construction s'appelle pTG1833.

La cassette d'expression de l'hirudine a été récupérée sous forme de fragment PstI-BglII pour être introduite dans un dérivé du phage M13, le M13TG131 (Kieny et al. 1983), pour y effectuer les mutagénèses ultérieures.

Une mutagénèse a été effectuée dans la séquence pre-pro pour créer un site HindIII qui permettra la fusion des séquences codantes de l'hirudine et l'échange aisé de celles-ci avec les diverses séquences mutées, tout en préservant la phase de lecture et en assurant la maturation correcte de la protéine synthétisée.

La séquence mutée est la suivante :

←——— séquence pre-pro modifiée ———→ | ←— Hirudine —→

......Glu Ala Val Ser Leu Asp Lys Arg Ile Thr......
......GAA GGG GTA AGC TTG GAT AAA AGA ATT ACG......
HindIII

Une mutation a été introduite dans la séquence codante de l'HV2 pour remplacer l'Asn⁴⁷ par une Lys⁴⁷. Cette mutagénèse a été effectuée avec l'oligonucléotide suivant :

5' CTTTCAGGTTTCGGTGTAC 3'

Le dérivé du phage M13 portant la cassette d'expression avec ces 2 mutations, appelé M13TG1945, servira de matrice à toutes les mutagénèses suivantes.

Pour induire de nouvelles mutations dans la séquence codante de HV2, les oligonucléotides suivants ont été utilisés :

TG1152 : 5' CTAATGGAACTGGCAACCA 3'
TG1153 : 5' TGGGTTCTAGAGGAGACAGCAACCAATG 3'
TG1154 : 5' CAGAAGAAGATTTACAATG 3'
TG1155 : 5' GATAAAAGAGTTGTTTATACAGACT 3'
TG1156 : 5' TATTTACAACCATAAATGAAAGAA 3'

Les séquences mutées correspondent respectivement aux variants suivants :
HV2 (Lys⁴⁷, Thr³⁵)
HV2 (Lys⁴⁷, Arg³³, Asp³⁵, Ser³⁶)
HV2 (Lys⁴⁷, Asp⁶³)
HV2 (Lys⁴⁷, Val¹, Val²)
HV2 (Lys⁴⁷, Pro⁶⁶)

La mutagénèse a été effectuée dans les conditions suivantes :
120 picomoles de chaque oligonucléotide ont été phosphorylés dans 100 µl de milieu réactionnel et environ 20 picomoles d'oligonucleotide phosphorylé ont été hybridés avec 1 picomole d'ADN simple brin du phage M13TG1919 dans 25 microlitres de tampon d'hybridation.
Après hybridation, le mélange d'ADNs a été soumis à l'action de la polymérase de Klenow et à la ligase du phage T4. Chaque mélange ainsi traité a servi à transfecter la souche de E. coli 71/18 (mut L) sur tapis de bactéries indicatrices JM103 (Messing et al. 1981). Les cellules infectées sont repérables parce qu'elles forment des plages à croissance plus lente ; les colonies ont été repiquées sur milieu complet puis transférées sur papier Whatman 540 en vue d'un criblage des cellules présentant les phages mutés.

L'identification des phages portant la séquence mutée est effectuée par hybridation in situ avec l'oligonucléotide qui a été utilisé pour la mutagénèse.
Les phages suivants, portant les séquences mutées ont été obtenus :
M13TG1946 → HV2 (Lys⁴⁷, Thr³⁵)
M13TG1947 → HV2 (Lys⁴⁷, Arg³³, Asp³⁵, Ser³⁶)
M13TG1948 → HV2 (Lys⁴⁷, Asp⁶³)
M13TG1949 → HV2 (Lys⁴⁷, Val¹, Val²)
M13TG1950 → HV2 (Lys⁴⁷, Pro⁶⁶)

### Exemple 2 Transfert des gènes mutés dans un vecteur d'expression-sécrétion et transformation des levures-hôtes.

Le vecteur d'expression-sécrétion pTG2835 comprend les éléments suivants :
1. L'origine de réplication et le gène de résistance à l'ampicilline du plasmide pBR322, permettant la multiplication du plasmide et sa sélection dans E. coli.
2. L'origine de réplication du plasmide 2 µ de levure et le gène ura3 de levure comme marqueur de sélection dans une souche de levure ura3⁻.
3. Un autre marqueur de sélection, le gène leu2 de levure qui peut être utilisé pour complémenter un gène leu2 défectif de la souche hôte.
4. Le promoteur de transcription du gène de l'α-phéromone de levure et le terminateur de transcription du gène PGK de levure.
5. La séquence pre-pro du gène de l'α-phéromone de la levure, manipulée pour y créer un site HindIII permettant la fusion du gène de l'hirudine, en phase et de manière à assurer la maturation correcte de la protéine synthétisée.

Les séquences codantes de l'hirudine portant les mutations sélectionnées et portées par les phages M13TG1946, 1947, 1948, 1949 et 1950 ont été récupérées sous forme de fragments HindIII et réintroduites dans le vecteur pTG2835 pour donner les plasmides suivants :
pTG2982 → HV2 (Lys⁴⁷, Thr³⁵)
pTG2983 → HV2 (Lys⁴⁷, Asp⁶³)
pTG2988 → HV2 (Lys⁴⁷, Arg³³, Asp³⁵, Ser³⁶)
pTG2989 → HV2 (Lys⁴⁷, Val¹, Val²)
pTG2990 → HV2 (Lys⁴⁷, Pro⁶⁶)

Une souche de levure S. cerevisiae TGYlsp4 (matα ura3.251.373.38 his03.11.15) a été transformée par ces différents plasmides.

L'activité antithrombine des différents variants de l'hirudine produits par ces souches a été comparée (voir figure 2).
On a ensemencé 20 ml de culture (YNBG + casamino acides 0.5 %) à DO₆₆₀ 0.02 avec chaque souche. Après 48 h d'agitation à 30°C, les cultures sont centrifugées (5000 rpm, 5 min) et les surnageants sont dosés. Une culture de TGYlsp4 pTG881 (plasmide ne portant pas de séquence codant pour HV2) est utilisée comme contrôle. L'activité des surnageants bruts est mesurée par leur effet inhibiteur sur l'activité de la thrombine (activité protéolytique sur un substrat synthétique, le chromozyme TH - Boehringer Mannheim) Le pourcentage d'inhibition en fonction des volumes de surnageant est donné dans la figure 2.

Cette étude montre clairement que les modifications de la séquence de l'hirudine n'entrainent aucune perte d'activité antithrombine ni aucune perte de productivité dans la levure.

L'usage de ces nouvelles molécules in vivo, comme agent antithrombotique, peut donc être envisagé.

### REFERENCES

Chang, J.Y. FEBS Letters 164, 307-313 (1983).
Dodt, J., Mueller, H.P., Seemüller, U. and Chang, J.Y. FEBS Letters 165, 180-184 (1984).
Dodt, J., Seemüller, U., Mascheler, R. and Fritz, H. Biol. Chem. Hoppe-Seyler 366, 379-385 (1985).
Dodt, J., Machleidt, W., Seemüller, U., Maschler, R. and Fritz, H. Biol. Chem. Hoppe-Seyler 367, 80 3-811 (1986).
Harvey, R.P., Degryse, E., Stefani, L., Schamber, F., Cazenave, J.P., Courtney, M., Tolstoshev, P. and Lecocq,
J.P. Proc. Natl. Acad. Sci. USA 83, 1084-1088 (1986).
Kieny, M.P., Lathe, R. and Lecocq, J.P. Gène 26, 91-99 (1983).
Markwardt, F. Methods Enzymol. 19, 924-932 (1970).
Messing, J., Crea, R., Seeburg, P.H. Nucl. Acids Res. 9, 309 (1981).
Petersen, T.E., Roberts, H.R., Sottrup-Jensen, L. and Magnusson, S. Protides, Biol., Fluids, Proc. Colloq. 23, 145-149 (1976).
Ruggeri, Z.M., Houghten, R.A., Russell, S.R. and Zimmerman, T.S. Proc. Natl. Acad. Sci. USA 83, 5708-5712 (1986).
Stone, S.R. and Hofsteenge, J. Biochem. 25, 4622-4628 (1986).
Zoller, M.J. and Smith, M.N. Methods in Enzymol. 100, 469 (1983).

## Revendications

1. Variants de l'hirudine, caractérisés en ce que la structure peptidique de ces variants comporte, par rapport aux variants de base HV, une des modifications suivantes :
- dans au moins un site Asn-X-Y de HV où X est un acide aminé quelconque, Y est choisi parmi Ser ou Thr ;
- une séquence Arg-Gly-Asp-Ser remplace une séquence d'acides aminés ;
- au moins un acide aminé supplémentaire est ajouté à l'extrémité C-terminale ;
les variants de base HV sont choisis parmi :
HV1, HV2, HV3, HV2 (Val¹, Val²), HV1 (AA⁶³), HV2 (AA⁶³), HV3 (AA⁶⁴), HV2 (Val¹, Val², AA⁶³), HV2 (Lys⁴⁷), HV2 (Val¹, Val², Lys⁴⁷), HV2 (Lys⁴⁷, AA⁶³), HV2 (Val¹, Val², Lys⁴⁷, AA⁶³), et AA est choisi parmi Glu et Asp.

2. Variant selon la revendication 1, caractérisé en ce que HV est choisi parmi :
HV2 (Lys⁴⁷), HV2 (Lys⁴⁷, AA⁶³), HV2 (Val¹, Val², Lys⁴⁷) et HV2 (Val¹, Val², Lys⁴⁷, AA⁶³).

3. Variant selon la revendication 2, caractérisé en ce qu'il est choisi parmi :
HV (Thr³⁵) et HV (Ser³⁵).

4. Variant selon la revendication 3, caractérisé en ce qu'il est choisi parmi :
HV2 (Lys⁴⁷, Thr³⁵) et HV2 (Ser³⁵, Lys⁴⁷), HV2 (Val¹, Val², Thr³⁵, Lys⁴⁷) et HV2 (Val¹, Val², Ser³⁵, Lys⁴⁷).

5. Variant selon l'une des revendications 1 à 4, caractérisé en ce qu'il comporte une structure partielle (Arg³³, Asp³⁵, Ser³⁶).

6. Variant selon la revendication 1, caractérisé en ce qu'il s'agit de : HV2 (Lys⁴⁷, Arg³³, Asp³⁵, Ser³⁶).

7. Variant selon l'une des revendications 1 à 6, caractérisé en ce qu'il comporte un acide aminé supplémentaire à l'extrémité C-terminale.

8. Variant selon la revendication 7, caractérisé en ce que l'acide aminé supplémentaire C-terminal est Pro.

9. Variant selon la revendication 1, caractérisé en ce qu'il s'agit d'un composé HV2 (Lys⁴⁷, Pro⁶⁶).

10. Variant selon la revendication 1, choisi parmi :
HV2 (AA⁶³, Lys⁴⁷) et HV2 (Val¹, Val², AA⁶³, Lys⁴⁷) où AA est Glu ou Asp.

11. Variant selon l'une des revendications 1 à 10, caractérisé en ce qu'il est sulfaté.

12. Variant selon l'une des revendications 1 à 11, caractérisé en ce que la chaîne peptidique est glycosylée.

13. A titre d'inhibiteur de la thrombine, un variant selon l'une des revendications 1 à 12.

14. A titre d'agent anticoagulant, une composition contenant à titre de principe actif au moins un variant selon l'une des revendications 1 à 12.

15. A titre de médicament, un variant selon l'une des revendications 1 à 12.

16. Procédé de fabrication d'un variant selon l'une des revendications 1 à 12 caractérisé en ce qu'il comporte des étapes de synthèse, d'hémisynthèse et/ou de manipulation génétique.

17. Procédé selon la revendication 16, caractérisé en ce qu'il comporte la fermentation d'une levure préalablement transformée soit par un plasmide comportant :
- l'origine de réplication du plasmide de levure 2 µ,
- le gène ura3,
- une origine de réplication dans E. coli et un marqueur de résistance à un antibiotique,
- un promoteur de transcription, la séquence "leader" et la séquence pre-pro du précurseur du facteur alpha, fusionnée en phase, en amont de la séquence codante du variant de l'hirudine,
- le terminateur de transcription du gène PGK de la levure qui sera placé en aval du gène dudit variant,
soit par un bloc fonctionnel d'ADN codant pour ledit variant de l'hirudine.

## Claims

1. Hirudin variants, wherein the peptide structure of these variants contains, relative to the parent HV variants, one of the following modifications:
- in at least one site Asn-X-Y of HV where X is any amino acid, Y is chosen from Ser or Thr;
- a sequence Arg-Gly-Asp-Ser replaces an amino acid sequence;
- at least one additional amino acid is added at the C-terminal end;
the parent HV variants are chosen from:
HV1, HV2, HV3, HV2 (Val¹, Val²), HV1 (AA⁶³), HV2 (AA⁶³), HV3 (AA⁶⁴), HV2 (Val¹, Val², AA⁶³), HV2 (Lys⁴⁷), HV2 (Val¹, Val², Lys⁴⁷) , HV2 (Lys⁴⁷, AA⁶³) , HV2 (Val¹, Val², Lys⁴⁷, AA⁶³) , and AA is chosen from Glu and Asp.

2. A variant as claimed in claim 1, wherein HV is chosen from :
HV2 (Lys⁴⁷), HV2 (Lys⁴⁷, AA⁶³), HV2 (Val¹, Val², Lys⁴⁷) and HV2 (Val¹, Val², Lys⁴⁷ AA⁶³).

3. The variant as claimed in claim 2, which is chosen from:
HV (Thr³⁵) and HV (Ser³⁵) .

4. The variant as claimed in claim 3, which is chosen from:
HV2 (Lys⁴⁷, Thr³⁵) and HV2 (Ser³⁵), Lys⁴⁷), HV2 (Val¹, Val², Thr³⁵, Lys⁴⁷) and HV2 (Val¹, Val², Ser³⁵, Lys⁴⁷).

5. The variant as claimed in one of claims 1 to 4, which contains a partial structure (Arg³³, Asp³⁵, Ser³⁶).

6. The variant as claimed in claim 1, the variant in question being: HV2 (Lys⁴⁷, Arg³³, Asp³⁵, Ser³⁶).

7. The variant as claimed in one of claim 1 to 6, which contains an additional amino acid at the C-terminal end.

8. The variant as claimed in claim 7, wherein the C-terminal additional amino acid is Pro.

9. The variant as claimed in claim 1, the variant in question being a compound HV2 (Lys⁴⁷, Pro⁶⁶).

10. The variant as claimed in claim 1, chosen from: HV2 (AA⁶³, Lys⁴⁷) and HV2 (Val¹, Val², AA⁶³, Lys⁴⁷), where AA is Glu or Asp.

11. The variant as claimed in one of claims 1 to 10, which is sulfated.

12. The variant as claimed in one of claims 1 to 11, wherein the peptide chain is glycosylated.

13. By way of a thrombin inhibitor, a variant as claimed in one of claims 1 to 12.

14. By way of an anticoagulant agent, a composition containing, by way of active principle, at least one variant as claimed in one of claims 1 to 12.

15. By way of a medicinal product, a variant as claimed in one of claims 1 to 12.

16. A method for manufacturing a variant as claimed in one of claims 1 to 12, which comprises stages of synthesis, partial synthesis and/or gene manipulation.

17. The method as claimed in claim 16, which comprises the fermentation of a yeast previously transformed either with a plasmid containing:
- the origin of replication of the yeast to 2 µ plasmid,
- the ura3 gene,
- an origin of replication in E. coli and a marker for resistance to an antibiotic,
- a transcription promoter, the leader sequence and the prepro sequence for the precursor of the alpha factor, fused in phase upstream from the coding sequence for the hirudin variant,
- the transcription terminator of the yeast PGK gene, which will be placed downstream from the gene for the said variant,
or with a functional DNA block coding for the said hirudin variant.

## Patentansprüche

1. Hirudin-Varianten, dadurch gekennzeichnet, daß die Peptid-Struktur dieser Varianten, bezogen auf die HV-Basen-Varianten, eine der folgenden Modifikationen aufweist:
- in mindestens einer Stelle Asn-X-Y von HV, worin X eine beliebige Aminosäure darstellt und Y ausgewählt wird aus Ser oder Thr;
- eine Sequenz Arg-Gly-Asp-Ser ersetzt eine Aminosäuresequenz;
- mindestens eine ergänzende Aminosäure ist an das C-terminale Ende gebunden;
wobei die HV-Basen-Varianten ausgewählt werden aus:
HV1, HV2, HV3, HV2 (Val¹, Val²), HV1 (AA⁶³), HV2 (AA⁶³), HV3 (AA⁶⁴), HV2 (Val¹, Val², AA⁶³), HV2 (Lys⁴⁷), HV2 (Val¹, val², Lys⁴⁷), HV2 (Lys⁴⁷, AA⁶³), HV2 (Val¹, Val², Lys⁴⁷, AA⁶³) und AA ausgewählt wird aus Glu und Asp.

2. Variante nach Anspruch 1, dadurch gekennzeichnet, daß HV ausgewählt wird aus:
HV2 (Lys⁴⁷), HV2 (Lys⁴⁷, AA⁶³), HV2 (Val¹, Val², Lys⁴⁷)und HV2 (Val¹, Val², Lys⁴⁷, AA⁶³).

3. Variante nach Anspruch 2, dadurch gekennzeichnet, daß es ausgewählt aus aus: HV (Thr³⁵) und HV (Ser³⁵).

4. Variante nach Anspruch 3, dadurch gekennzeichnet, daß sie ausgewählt wird aus:
HV2 (Lys⁴⁷, Thr³⁵) und HV2 (Ser³⁵, Lys⁴⁷), HV2 (Val¹, Val², Thr³⁵, Lys⁴⁷) und HV2 (Val¹, Val², Ser³⁵, Lys⁴⁷).

5. Variante nach einem der Ansprüche 1 bis 4, dadurch gekennzeichnet, daß sie eine Partialstruktur (Arg³³, Asp³⁵, Ser³⁶) aufweist.

6. Variante nach Anspruch 1, dadurch gekennzeichnet, daß es sich dabei handelt um HV2 (Lys⁴⁷, Arg³³, Asp³⁵, Ser³⁶)

7. Variante nach einem der Ansprüche 1 bis 6, dadurch gekennzeichnet, daß sie eine ergänzende Aminosäure an dem C-terminalen Ende aufweist.

8. Variante nach Anspruch 7, dadurch gekennzeichnet, daß es sich bei der ergänzenden C-terminalen Aminosäure um Pro handelt.

9. Variante nach Anspruch 1, dadurch gekennzeichnet, daß es sich um eine Verbindung HV2 (Lys⁴⁷, Pro⁶⁶) handelt.

10. Variante nach Anspruch 1, die ausgewählt wird aus:
HV2 (AA⁶³, Lys⁴⁷) und HV2 (Val¹, Val², AA⁶³, Lys⁴⁷) worin AA für Glu oder Asp steht.

11. Variante nach einem der Ansprüche 1 bis 10, dadurch gekennzeichnet, daß sie sulfatiert ist.

12. Variante nach einem der Ansprüche 1 bis 11, dadurch gekennzeichnet, daß die Peptidkette glycosyliert ist.

13. Variante nach einem der Ansprüche 1 bis 12 als Thrombin-Inhibitor.

14. Zusammensetzung, die als aktives Prinzip (Wirkstoff) mindestens eine Variante nach einem der Ansprüche 1 bis 12 enthält, als Antikoagulans.

15. Variante nach einem der Ansprüche 1 bis 12 als Arzneimittel.

16. Verfahren zur Herstellung einer Variante nach einem der Ansprüche 1 bis 12, dadurch gekennzeichnet, daß es Synthese-, Hemisynthese- und/oder genetische Manipulations-Stufen umfaßt.

17. Verfahren nach Anspruch 16, dadurch gekennzeichnet, daß es umfaßt die Fermentation einer Hefe, die vorher transformiert worden ist, entweder durch ein Plasmid, das aufweist:
- den Replikationsursprung des Hefe-Plasmids 2 µ,
- das Gen ura3,
- einen Replikationsursprung in E. coli und einen Marker für die Resistenz gegenüber einem Antibiotikum,
- einen Transkriptions-Promotor, die "Leader"-Sequenz und die Sequenz pre-pro des Vorläufers des α-Faktors, die in Phase fusioniert sind, stromaufwärts von der codierenden Sequenz der Hirudin-Variante,
- den Transkriptions-Terminator des Gens PGK der Hefe, der stromabwärts von dem Gen der genannten Variante angeordnet ist,
oder durch einen funktionellen DNA-Block, der für die genannte Hirudinvariante codiert.
